# EUROPEAN PATENT APPLICATION

(11) **EP 4 464 234 A1**
(43) Date of publication of application: **20.11.2024**
(21) Application number: 23774387.7
(22) Date of filing: 27.02.2023
(51) Int. Cl.: A61B 1/24, A61B 1/00

(54) **STANDARD-IMAGING ASSISTANCE DEVICE**

(30) Priority: 25.03.2022 JP 2022050888
(71) Applicant: The Yoshida Dental Mfg. Co., Ltd., Sumida-ku Tokyo 130-8516 (JP)
(72) Inventor: ISHIWATA, Masahiro, Tokyo 130-8516 (JP)
(74) Representative: Simmons & Simmons LLP (Munich)
(86) International application number: PCT/JP2023/006974
(87) International publication number: WO 2023/181788

(57) **Abstract**

A standard-imaging assistance device (1) assists standard imaging performed by a user and is provided with a standard-imaging imaging mode for imaging the oral cavity of an examinee. The standard-imaging assistance device comprises: an operation unit (8) that receives a setting operation for an imaging mode from the user; and a control unit (20) that, according to the setting operation received by the operation unit (8) and by using an imaging unit, performs imaging of a plurality of specified imaging sites within the oral cavity of the same examinee, and executes a standard imaging mode in which the aforementioned captured images are respectively associated.

## Description

### TECHNICAL FIELD

The present invention relates to a standard-imaging assistance device and particularly relates to a standard-imaging assistance device which assists standard imaging in the oral cavity.

### BACKGROUND ART

In dental examination, intraoral photographs of patients, examined subjects, and the like (hereinafter, these are also collectively referred to as "examination subjects") are sometimes captured. Intraoral photographs refer to photographs obtained by imaging the front faces of teeth, the alignment of the teeth, the state of the gingiva, and the like of an examination subject by using a camera. An intraoral photograph is normally composed of a plurality of captured images such as five captured images or nine captured images, for example. Such intraoral photographs have not only an aspect as recorded matters for contents of diagnosis or case reports, and the like, but also an aspect as communication tools used for the purpose of explaining treatment strategies to patients, sharing the states of oral cavities of patients among clinics, and the like. Among a plurality of captured images included in an intraoral photograph, at least one of various imaging conditions (for example, the distance between a camera and an imaging subject, the resolution, the magnification factor, and the like) is required to be standardized or substantially standardized, that is, standardization is required.

In standard imaging of an intraoral photograph, imaging is conducted by using a camera such as a single-lens reflex camera in a state where teeth which need to be photographed are set in each frame of a plurality of captured images included in a standard-image photograph. This standard imaging employs an imaging method in which the shutter button of the camera is pressed when the teeth of the imaging subject are taken into a frame. By two-dimensionally arranging a plurality of captured images captured in this way (specifically, by arranging the plurality of captured images in accordance with a so-called five-image method or nine-image method), the dentist grasps the state in the oral cavity of the examination subject. Standard-image photographs are utilized, for example, for follow-up of the conditions before and after the treatment in the oral cavity of the examination subject, and the like.

Note that although not standard imaging, a practice is known in which a dentist captures images in the oral cavity of a patient by using an intraoral camera and conducts medical examination while observing captured images displayed on a monitor (Patent Literature 1).

### CITATION LIST

### Patent Literature

Patent Literature 1: Japanese Patent Application Publication No. 2005-103048

### SUMMARY OF INVENTION

### Technical Problem

In the conventional standard imaging, it is necessary for the user to check captured images obtained respectively by capturing images of a plurality of image-capture sites specified in the oral cavity of the examination subject, determine for which image-capture site each captured image is, and collect a plurality of captured images of the same examination subject. For this reason, there has been a demand for a technique that can facilitate standard imaging.

The present invention has been made in view of the above-described circumstances, and an object thereof is to provide a standard-imaging assistance device that can facilitate standard imaging.

### Solution to Problem

To solve the above-described problem, a standard-imaging assistance device according to the present invention is a standard-imaging assistance device which includes an imaging mode of standard imaging to image an inside of an oral cavity of an examination subject and which assists the standard imaging conducted by a user, comprising: an operating part which receives a setting operation of an imaging mode from the user; and a controller which captures images of a plurality of image-capture sites specified in the oral cavity of the same examination subject by using an image capturer in accordance with the setting operation received in the operating part and executes a standard-imaging mode which associates the captured images thus captured with one another.

### Advantageous Effects of Invention

The present invention can facilitate standard imaging.

### BRIEF DESCRIPTION OF DRAWINGS

FIG. 1 is a block diagram schematically showing a standard-imaging assistance device according to a first embodiment of the present invention.
FIG. 2A is an appearance diagram of the standard-imaging assistance device of FIG. 1 as viewed from a front face. FIG. 2B is a schematic diagram of the standard-imaging assistance device of FIG. 1 as viewed from a side.
FIG. 3 is a schematic diagram showing a method for imaging an upper jaw using the standard-imaging assistance device of the FIG. 1.
FIG. 4 is a schematic diagram of correspondence relation between dental formula numbers and a dentition of an image-capture subject.
FIG. 5A is a schematic diagram showing correspondence between image-capture sites of a five-image method and dental formula numbers. FIG. 5B is a schematic diagram showing correspondence between image-capture sites of a nine-image method and dental formula numbers.
FIG. 6A is a schematic diagram showing names of the image-capture sites of the five-image method. FIG. 6B is a schematic diagram showing names of the image-capture sites of the nine-image method.
FIG. 7A is an example of screen display of standard-imaging mode buttons. FIG. 7B is an example of screen display of acquisition count input buttons. FIG. 7C is an example of screen display of image-capture site setting buttons.
FIG. 8A is an example of screen display of buttons of a specific site setter of the five-image method. FIG. 8B is an example of screen display of buttons of a specific site setter of the nine-image method. FIG. 8C is an example of screen display of specific site information indication regions of the five-image method. FIG. 8D is an example of screen display of specific site information indication regions of the nine-image method.
FIG. 9A is a schematic diagram showing correspondence between the image-capture sites of the five-image method and outputs of an orientation detection sensor. FIG. 9B is a schematic diagram showing correspondence between the image-capture sites of the nine-image method and outputs of the orientation detection sensor.
FIG. 10A is a schematic diagram showing correspondence between the image-capture sites of the five-image method and reverse processes. FIG. 10B is a schematic diagram showing correspondence between the image-capture sites of the nine-image method and reverse processes.
FIG. 11A is a schematic diagram showing correspondence between the image-capture sites of the five-image method and magnification factors. FIG. 11B is a schematic diagram showing correspondence between the image-capture sites of the nine-image method and magnification factors.
FIG. 12 is a flowchart showing a flow of processing of the standard-imaging mode of the standard-imaging assistance device according to the embodiment of the present invention.
FIG. 13 is a flowchart showing a flow of manual setting of an image-capture site of FIG. 12.
FIG. 14 is a flowchart showing a flow of automatic setting of an image-capture site of FIG. 12.
FIG. 15 is a block diagram schematically showing a standard-imaging assistance device according to a second embodiment of the present invention.
FIG. 16A is a graph showing correction values in accordance with distances. FIG. 16B is a regional magnification factor table.
FIG. 17A is a schematic diagram showing correspondence between the image-capture sites of the five-image method and correction values of a magnification factor. FIG. 17B is a schematic diagram showing correspondence between the image-capture sites of the nine-image method and correction values of a magnification factor.
FIG. 18A is captured images before a masking process. FIG. 18B is captured images after the masking process.

### DESCRIPTION OF EMBODIMENTS

Modes for carrying out a standard-imaging assistance device according to the present invention will be described in detail with reference to the drawings. Note that the sizes, positional relations, and the like of members shown in each drawing are sometimes exaggerated in order to clarify the description.

### (First Embodiment)

### [Configuration of Standard-Imaging Assistance Device]

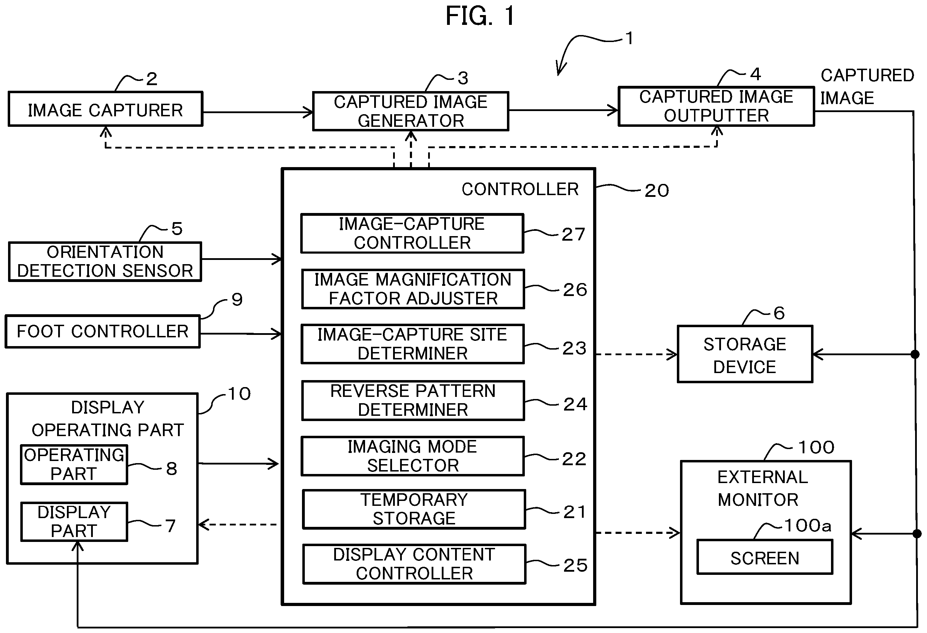

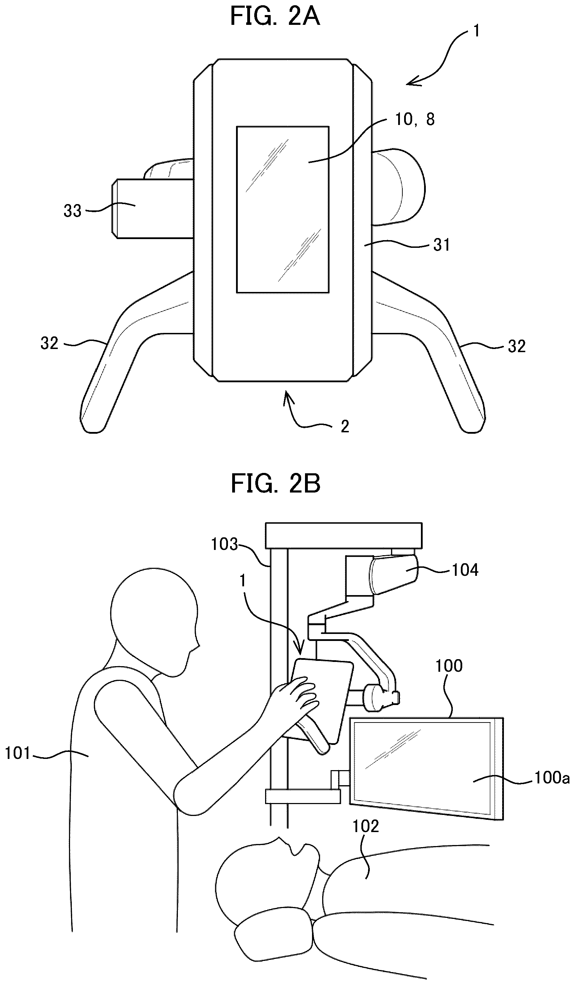

A standard-imaging assistance device 1 according to a first embodiment shown in FIG. 1 is a device which includes an imaging mode of standard imaging to image an oral cavity of an examination subject and which assists the standard imaging conducted by a user. The standard-imaging assistance device 1 includes an image capturer 2, a captured image generator 3, a captured image outputter 4, an orientation detection sensor 5, a storage device 6, a display part 7, an operating part 8, a foot controller 9, and a controller 20. The display part 7 and the operating part 8 may be integrated to configure a display operating part 10 including a touch panel. An example of the standard-imaging assistance device 1 including the display operating part 10 including a touch panel is shown in FIG. 2A and FIG. 2B. FIG. 2A is an appearance diagram of the standard-imaging assistance device 1 as viewed from a front side. As shown in FIG. 2A, the standard-imaging assistance device 1 includes a main body 31, grip parts 32, and an arm connector 33.

The main body 31 is a casing incorporating the image capturer 2, the captured image generator 3 and the captured image outputter 4 (hereinafter, these are collectively referred to simply as a camera), as well as the controller 20. On the front face of the main body 31, the display operating part 10 is formed, and on the bottom of the main body 31, a camera lens and a light for illuminating an imaging subject are provided in the state of facing downward. The display operating part 10 is, for example, a touch panel, and serves as the display part 7 and the operating part 8.

The grip parts 32 are curved bar-shaped handles which the user grips when moving the standard-imaging assistance device 1 to a desired position, and are provided on a lower portion on a left side face and a lower portion on a right side face of the main body 31. On the grip parts 32, various button operation switches for a main power supply, a camera power supply, a camera illumination, and the like are disposed.

The arm connector 33 is means for connecting and fixing the main body 31 of the standard-imaging assistance device 1 to a stand or a hoisting attachment at a predetermined height.

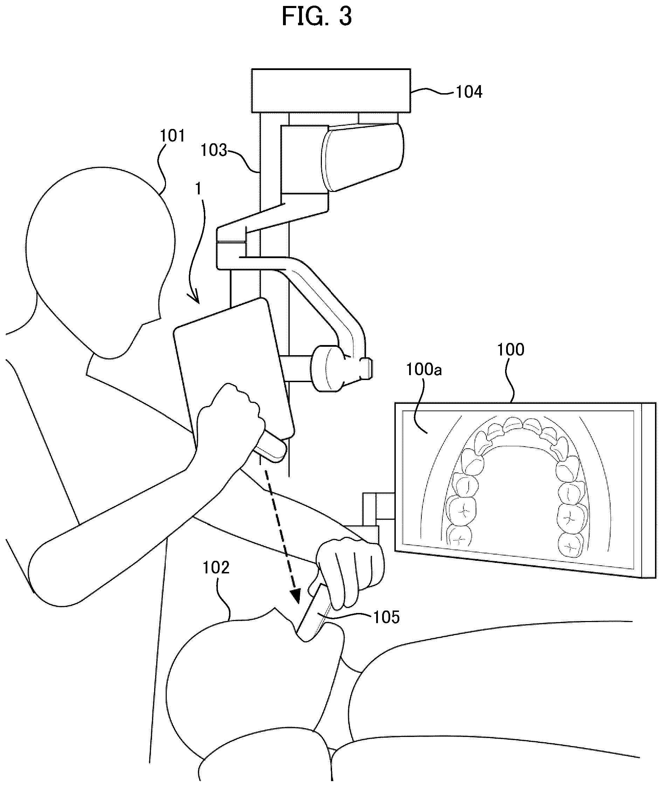

FIG. 2B is a schematic diagram of the standard-imaging assistance device 1 as viewed from a side and shows how imaging is conducted. The user 101 is a dentist, a dental hygienist, or the like. The examination subject 102 is asked to lie on a dental chair and open the mouth in a supine position. Here, the standard-imaging assistance device 1 is connected and fixed to the front end of an arm 104 extending from a stand 103 in a horizontal direction. The stand 103 is a stand independently provided upright or a pole fixed to a dental unit which is not shown. FIG. 3 is a schematic diagram showing a method for imaging the upper jaw using the standard-imaging assistance device 1. In this way, the user 101 uses a mirror 105 depending on an image-capture site and captures an image reflected in the mirror 105.

The image capturer 2 includes a camera lens and an imaging element. The imaging element is, for example, a CCD (Charge-Coupled Device) imaging element, a CMOS (Complementary MOS) imaging element, or the like. The image capturer 2 captures an image in accordance with a control signal from the controller 20. The image capturer 2 converts a captured image to an electrical signal (image signal) and outputs the image signal to the captured image generator 3.

The captured image generator 3 generates a captured image based on an image signal outputted from the image capturer 2. The captured image generator 3 outputs a captured image generated from an image signal to the captured image outputter 4.

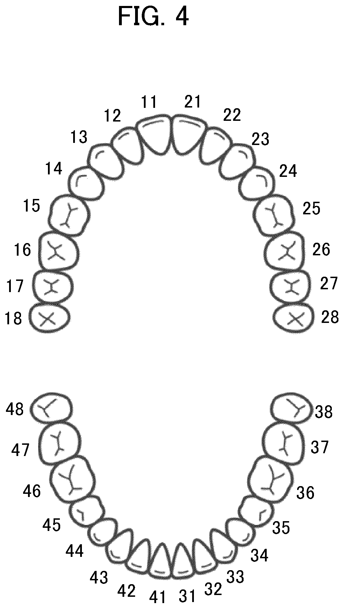

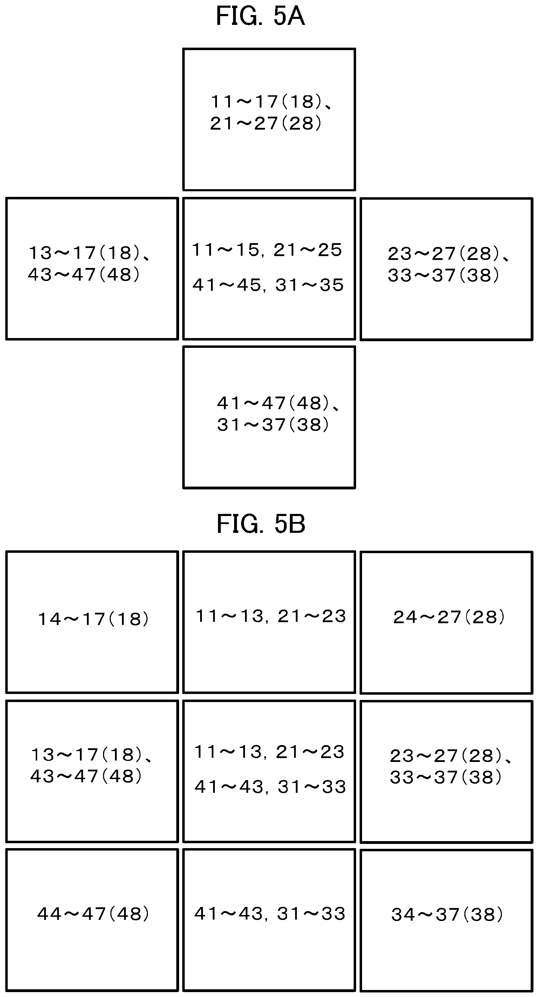

The captured image outputter 4 performs a reverse process on an acquired captured image and outputs the captured image to the display part 7, the external monitor 100, and the storage device 6. The captured image outputter 4 outputs data of the captured image on which the reverse process has been performed in a format containing information on an image-capture site of an image-capture subject from which the captured image has been acquired. The format containing information on the site of the image-capture subject is, for example, the DICOM format. DICOM stands for "Digital Imaging and Communications in Medicine" and is an international standard that specifies an image standard and a communication protocol of medical images. In addition, DICOM tag (0008,2228) represents the primary anatomic structure sequence. The captured image outputter 4 stores a standard-image photograph in the DICOM format and records dental formula numbers (see FIG. 4, FIG. 5A, and FIG. 5B) in the DICOM tag (0008,2228). From this dental formula number, the imaged site can be determined.

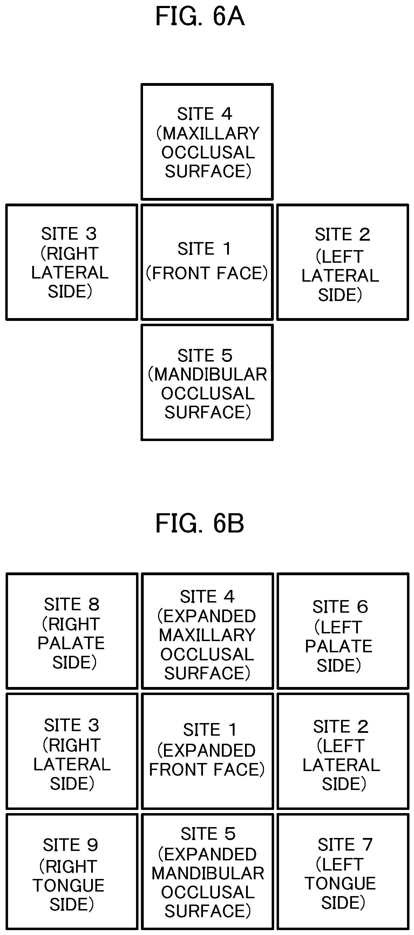

FIG. 4 shows correspondence relation between dental formula numbers and a dentition of an image-capture subject in the FDI System (two-digit system) as an example. FIG. 5A is a schematic diagram showing correspondence between image-capture sites of the five-image method and dental formula numbers. FIG. 5B is a schematic diagram showing correspondence between image-capture sites of the nine-image method and dental formula numbers. In the case of the five-image method, names of site 1 to site 5 shown in FIG. 6A are the front face, the left lateral side, the right lateral side, the maxillary occlusal surface, and the mandibular occlusal surface, respectively. In the case of the nine-image method, names of site 1 to site 9 shown in FIG. 6B are the front face, the left lateral side, the right lateral side, the expanded maxillary occlusal surface, the expanded mandibular occlusal surface, the left palate side, the left tongue side, the right palate side, and the right tongue side, respectively.

For example, in the case where the image-capture site of the image-capture subject is the front face of the five-image method, since the image of the front face is a captured image of teeth of dental formula numbers 11 to 15, 21 to 25, 41 to 45, and 31 to 35 as shown in FIG. 5A, the captured image outputter 4 records the following character string in the DICOM tag (0008,2228). "15¥14¥13¥12¥11¥21¥22¥23¥24¥25¥45¥44¥43¥42¥41¥31¥32¥33¥34¥35"

Although in the above example, all the dental formula numbers are listed, only some of the dental formula numbers such as 11 and 12 may be recorded. Note that not only the FDI System but also the American System (Universal System) or the Zsigmondy System (Zsigmondy & Palmer System) may be employed instead.

The orientation detection sensor 5 detects a three-dimensional orientation of the image capturer 2 relative to the reference orientation of the image capturer 2. As the orientation detection sensor 5, any of detectors of various methods to detect a three-dimensional orientation may be used. The orientation detection sensor 5 may be, for example, a gyroscope sensor, an acceleration sensor, a geomagnetic sensor, or the like as long as the orientation detection sensor 5 can detect a three-axis orientation. In addition, the orientation detection sensor 5 may be configured by combining, for example, a gyroscope sensor and an acceleration sensor. The orientation detection sensor 5 may be configured with a single semiconductor chip. The orientation detection sensor 5 is disposed, for example, near the image capturer 2. Note that the position of the orientation detection sensor 5 is not limited to near the image capturer 2 as long as the orientation detection sensor 5 can detect the orientation of the image capturer 2. The orientation detected by the orientation detection sensor 5 is expressed by three-dimensional angles. The orientation detection sensor 5 outputs the detected angles to the controller 20.

The storage device 6 stores data of a captured image outputted from the captured image outputter 4. The storage device 6 is an image memory. The storage device 6 may be, for example, storage means (HDD) for general personal computers, a HDD to be externally connected, or an image storage server connected to the standard-imaging assistance device 1 via a network, or the like.

The display part 7 displays a captured image outputted from the captured image outputter 4. The display part 7 is configured with, for example, a liquid-crystal display panel or the like.

The operating part 8 receives a setting operation of the imaging mode from the user. The operating part 8 has buttons with which it is possible to input an instruction through the operation of the user. Here, the operating part 8 having buttons obviously means that, for example, in the case where the display operating part 10 includes a touch panel, the operating part 8 includes buttons when buttons are displayed on the screen. Moreover, the touch panel can display the buttons even when the display of the button is deactivated from the screen, and it can be said that the touch panel substantially includes the buttons even if the display of the buttons has been deactivated.

Note that the buttons of the operating part 8 may be, for example, a mechanical switch (including a sheet switch) instead of the buttons displayed on the touch panel.

Hereinafter, the description is made on the premise that the operating part 8 is the same meaning as the display operating part 10 including a touch panel as an example.

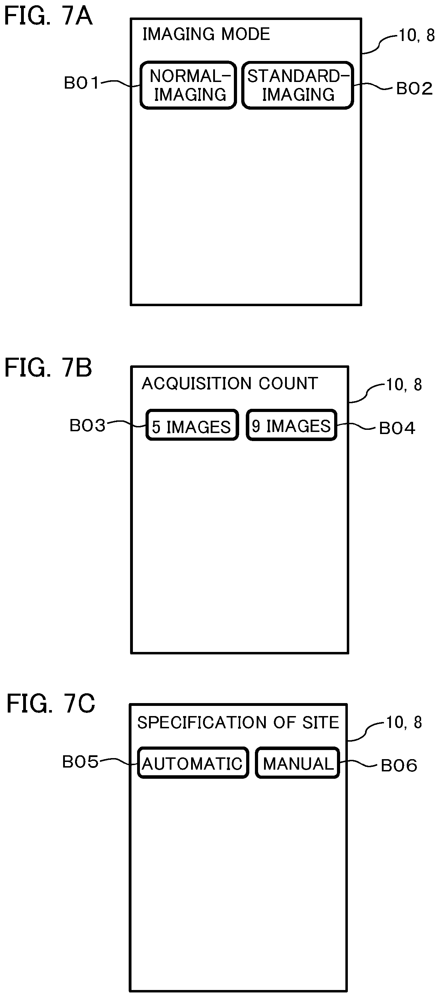

FIG. 7A to FIG. 7C are examples of buttons displayed on the display operating part 10 (the operating part 8) including a touch panel. As shown in FIG. 7A, the display operating part 10 has a standard-imaging mode button B02. The standard-imaging mode button B02 is a button with which it is possible to input a standard-imaging mode execution instruction for executing a standard-imaging mode. Here, on the screen of the display operating part 10, a normal-imaging mode button B01 and the standard-imaging mode button B02 are simultaneously displayed. Note that the normal-imaging mode button B01 is a button with which it is possible to select normal imaging other than the standard imaging. The normal imaging is, for example, imaging of a root canal, imaging of a treatment site of one tooth, or the like.

As shown in FIG. 7B, the display operating part 10 has acquisition count input buttons B03 and B04. The acquisition count input buttons B03 and B04 are buttons with which it is possible to input whether to execute a five-image method acquiring mode for obtaining captured images of the five-image method of standard imaging or a nine-image method acquiring mode for obtaining captured images of the nine-image method of the standard imaging. Here, the acquisition count input button B03 is a button for selecting standard imaging in the five-image method, and the acquisition count input button B04 is a button for selecting standard imaging in the nine-image method.

As shown in FIG. 7C, the display operating part 10 has image-capture site setting buttons B05 and B06. The image-capture site setting buttons B05 and B06 are buttons with which it is possible to input whether to automatically set or manually set which image-capture site of a plurality of image-capture sites specified in the oral cavity of the examination subject, the image-capture site of the captured image to be acquired by the standard-imaging assistance device 1 is. Here, the image-capture site setting button B05 is a button for selecting a method for automatic setting an image-capture site in accordance with a result of detecting the orientation of the image capturer 2 by the orientation detection sensor 5. The image-capture site setting button B06 is a button for selecting a method for the user to specify (manually set) an image-capture site.

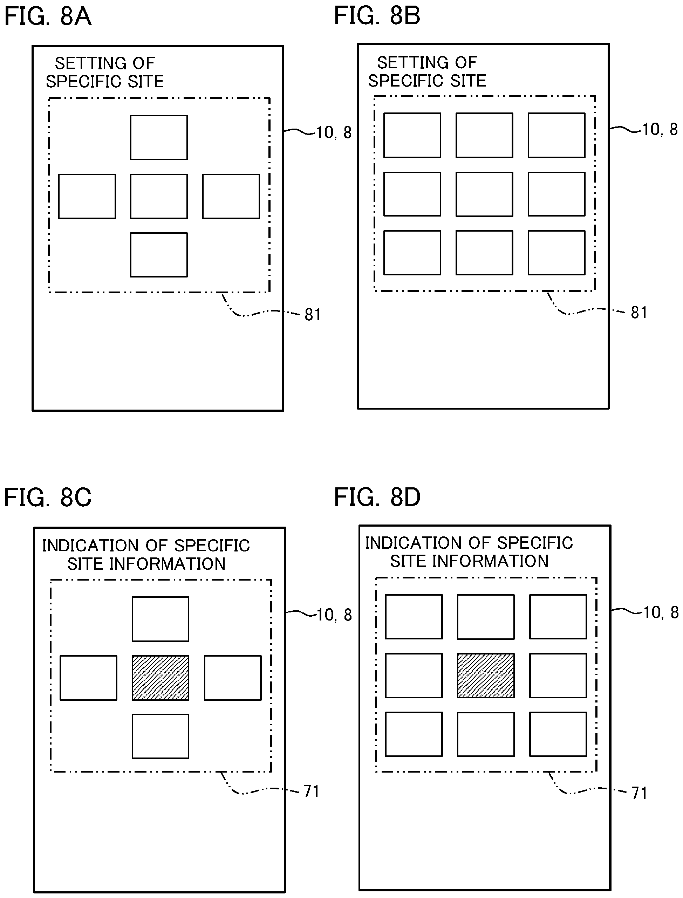

As shown in FIG. 8A and FIG. 8B, the display operating part 10 includes a specific site setter 81. The specific site setter 81 is a setter with which it is possible to input, through the operation of the user, which image-capture site of a plurality of image-capture sites specified in the oral cavity of the examination subject, an image-capture site of a captured image to be acquired by the standard-imaging assistance device 1 is. The specific site setter 81 is composed of five buttons corresponding respectively to the image-capture sites of the five-image method of the standard imaging as shown in FIG. 8A, and these five buttons are arranged in a cross shape. These five buttons only have to be arranged in a cross shape as viewed from the user. Here, the five buttons are arranged two-dimensionally in a cross shape on the display operating part 10, for example.

In addition, the specific site setter 81 is composed of nine buttons corresponding respectively to the image-capture sites of the nine-image method of the standard imaging as shown in FIG. 8B, and these nine buttons are arranged two-dimensionally in a matrix shape of vertical three columns and horizontal three rows on the display operating part 10, for example. The buttons included in the specific site setter 81 are specific site correspondence image information corresponding respectively to the plurality of image-capture sites, and are displayed on the display operating part 10 (display part 7), which displays a captured image to be acquired by the standard-imaging assistance device 1 or a captured image acquired by the standard-imaging assistance device 1.

As shown in FIG. 8C and FIG. 8D, the display operating part 10 includes a specific site information indication region 71. The specific site information indication region 71 is a region for indicating a captured image to be acquired by the standard-imaging assistance device 1 or a region for indicating which image-capture site of the plurality of image-capture sites specified in the oral cavity of the examination subject, an image-capture site of the captured image is.

In FIG. 8C and FIG. 8D, the regions included in the specific site information indication region 71 are image information corresponding respectively to the plurality of image-capture sites, and are displayed on the display part 7, which displays a captured image to be acquired by the standard-imaging assistance device 1 or a captured image acquired by the standard-imaging assistance device 1.

The specific site information indication region 71 is composed of five regions corresponding respectively to the image-capture sites of the five-image method of the standard imaging as shown in FIG. 8C, and these five regions are arranged two-dimensionally in a cross shape on the display operating part 10, for example. FIG. 8C is an example in which the specific site information indication region 71 identifiably indicates that the image-capture site to be acquired by the standard-imaging assistance device 1 is the image-capture site of the front face. The specific site information indication region 71 may allow a specific region to be identified by changing the displayed color, increasing the light intensity, or blinking, of the specific region, for example.

The specific site information indication region 71 is composed of nine regions corresponding respectively to the image-capture sites of the nine-image method of the standard imaging as shown in FIG. 8D, and these nine regions are arranged two-dimensionally in a matrix shape of vertical three columns and horizontal three rows on the display operating part 10, for example. Note that FIG. 8D is also an example in which the specific site information indication region 71 identifiably indicates that the image-capture site to be acquired by the standard-imaging assistance device 1 is the image-capture site of the front face.

The foot controller 9 inputs a predetermined instruction to the standard-imaging assistance device 1. The foot controller 9 includes a pedal, and an instruction of starting imaging may be inputted by the user stepping down the pedal, for example. The foot controller 9 may input instructions of selecting and determining image-capture sites, for example. The foot controller 9 can be operated without using hands and is thus hygienic.

The controller 20 executes the standard-imaging mode. The controller 20 includes a temporary storage 21, an imaging mode selector 22, an image-capture site determiner 23, a reverse pattern determiner 24, a display content controller 25, an image magnification factor adjuster 26, and an image-capture controller 27, as shown in FIG. 1. The temporary storage 21 temporarily stores calculation results obtained by calculation of each unit of the controller 20, and is, for example, a RAM (Random Access Memory) or the like.

The imaging mode selector 22 conducts processing of determining the operation of setting the imaging mode received at the operating part 8 and processing of causing the display operating part 10 to display various correspondence image information corresponding to the determination processing. The imaging mode selector 22 causes the display operating part 10 to display, for example, the normal-imaging mode button B01, the standard-imaging mode button B02, the acquisition count input buttons B03 and B04, the image-capture site setting buttons B05 and B06, the specific site setter 81, and the like. In addition, in the case where information of the image-capture site the image of which is to be captured is indicated from the image-capture site determiner 23, the imaging mode selector 22 causes the display operating part 10 to identifiably display a region corresponding to the image-capture site in the specific site information indication region 71.

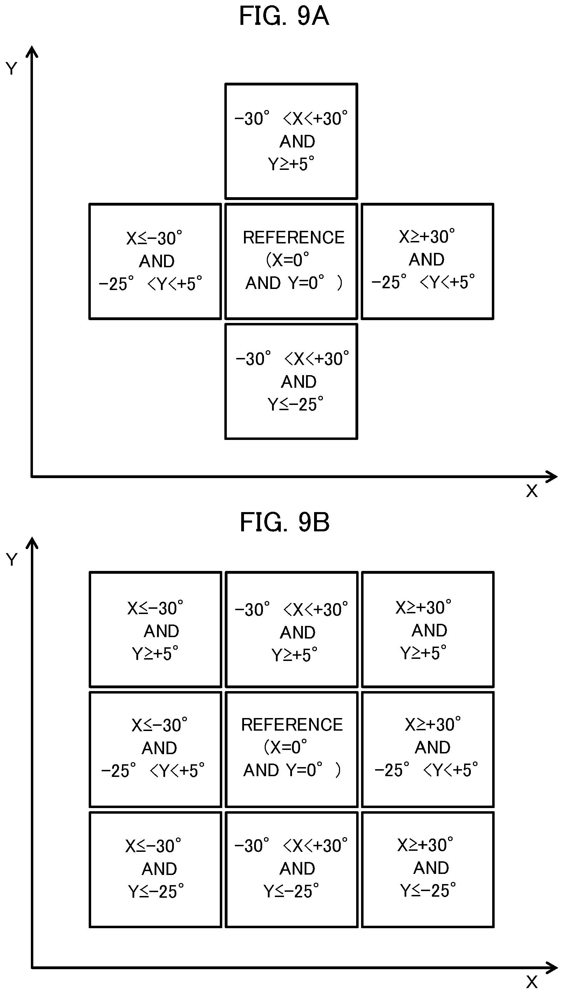

The image-capture site determiner 23 determines which image-capture site of the plurality of image-capture sites specified in the oral cavity of the examination subject, the image-capture site of the captured image to be acquired by the standard-imaging assistance device 1 is, based on a result of detection of the orientation detection sensor 5. The image-capture site determiner 23 determines a site an image of which is to be captured, based on the result of detection of the orientation detection sensor 5 and predetermined determination conditions for every imaging. For example, determination conditions shown in FIG. 9A and FIG. 9B may be employed. FIG. 9A shows determination conditions of the five-image method, and FIG. 9B shows determination conditions of the nine-image method.

Normally, since the site 1 (front face) is initially imaged, the result of detection of the orientation detection sensor 5 at this time is set as a reference orientation of the image capturer 2 (orientation detection reference position; X=0°, Y=0°). In the following imaging, the image of which site is to be captured can be identified from a three-dimensional orientation relative to the reference orientation (difference of an output from the orientation detection sensor 5).

In the case where the difference of the output from orientation detection sensor 5 is X≥+30° and -25°<Y<+5°, the image-capture site determiner 23 determines that the image-capture site is the site 2. In this case, the image-capture site determiner 23 indicates the result of determination of the image-capture site (information that the site the image of which is to be captured is the site 2) to the imaging mode selector 22.

In the case where the difference of the output from the orientation detection sensor 5 is X≤-30° and -25°<Y<+5°, the image-capture site determiner 23 determines that the image-capture site is the site 3.

In the case where the difference of the output from the orientation detection sensor 5 is - 30°<X<+30° and Y≥+5°, the image-capture site determiner 23 determines that the image-capture site is the site 4.

In the case where the difference of the output from the orientation detection sensor 5 is - 30°<X<+30° and Y≤-25°, the image-capture site determiner 23 determines that the image-capture site is the site 5.

In the case where the difference of the output from the orientation detection sensor 5 is X≥+30° and Y≥+5°, the image-capture site determiner 23 determines that the image-capture site is the site 6.

In the case where the difference of the output from the orientation detection sensor 5 is X≥+30° and Y≤-25°, the image-capture site determiner 23 determines that the image-capture site is the site 7.

In the case where the difference of the output from the orientation detection sensor 5 is X≤-30° and Y≥+5°, the image-capture site determiner 23 determines that the image-capture site is the site 8.

In the case where the difference of the output from the orientation detection sensor 5 is X≤-30° and Y≤-25°, the image-capture site determiner 23 determines that the image-capture site is the site 9.

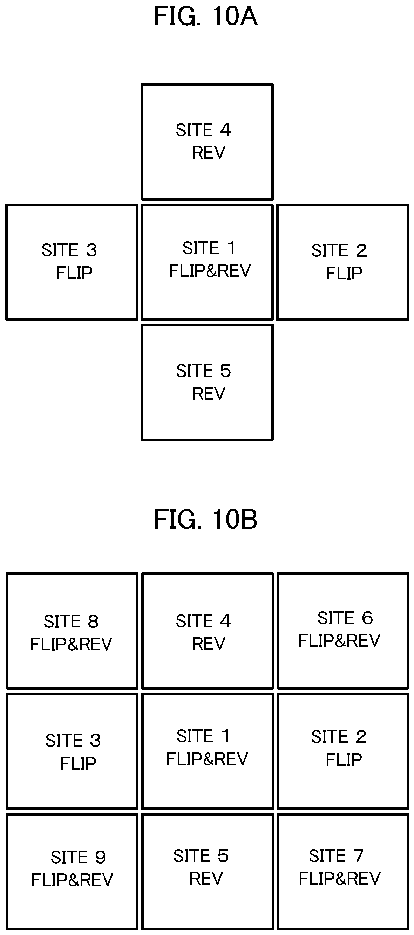

The reverse pattern determiner 24 determines which of a plurality of reverse processes determined in advance, a reverse process to be performed on a captured image acquired from any image-capture site as an image-capture subject is for every imaging of the five-image method or the nine-image method. As the plurality of reverse processes, for example, correspondence tables shown in FIG. 10A and FIG. 10B may be employed. FIG. 10A shows a correspondence table of the five-image method, and FIG. 10B shows a correspondence table of the nine-image method. In each correspondence table, FLIP indicates a vertical reverse process, and REV indicates a lateral reverse process.

In the case where the site 1 (front face) in the oral cavity has been selected in the specific site setter 81 of the display operating part 10 through the operation of the user, the reverse pattern determiner 24 determines that vertical and lateral reverse processes (FLIP&REV) are necessary for a captured image of the selected image-capture site.

In the case where the result of determination of the image-capture site determiner 23 specifies the site 2 (left lateral side) in the oral cavity, the reverse pattern determiner 24 determines that a vertical reverse process (FLIP) is necessary for a captured image of the specified image-capture site.

The reverse pattern determiner 24 outputs the result of the determination (the content of the reverse process of the image-capture site or information specifying the content of the reverse process) to the captured image outputter 4, for example, via the image-capture controller 27 or directly. The captured image outputter 4 performs the reverse process on the acquired captured image based on the result of the determination of the reverse pattern determiner 24 and outputs the captured image on which the reverse process has been performed. The captured image outputter 4 outputs the captured image on which the reverse process has been performed to the display operating part 10, the external monitor 100, and the storage device 6. Note that although there could be a case where an image-capture site for which a reverse process is unnecessary is generated depending on an imaging method, in the embodiment, the reverse processes are deemed to encompass such cases where there is an image-capture site for which no reverse process is performed.

The display content controller 25 controls the content of the display on the screen of the display operating part 10. The display content controller 25 replaces a captured image with respect to a specific image-capture site acquired by the image capturer 2 with specific site correspondence image information corresponding to the specific image-capture site and causes the display operating part 10 to display the specific site correspondence image information. Here, the specific site correspondence image information means an image of a button indicating the image-capture site of, for example, the front face in the oral cavity, or the like. Note that the display content controller 25 acquires a captured image outputted from the captured image outputter 4. This captured image to be acquired is a captured image with respect to a specific image-capture site of the image-capture subject.

In the present embodiment, the standard-imaging assistance device 1 is connected to the external monitor 100, and the display content controller 25 can also control the content of the display on a screen 100a of the external monitor 100 connected to the standard-imaging assistance device 1. In this case, the display content controller 25 replaces a captured image with respect to a specific image-capture site acquired from the image capturer 2 with specific site correspondence image information corresponding to the specific image-capture site and causes the screen 100a of the external monitor 100 to display the specific site correspondence image information. The external monitor 100 is a large-size monitor having a high image quality. The external monitor 100 is preferably a 4K or more, 50-inch or more monitor, for example. With this configuration, the user can more easily see the content of the display on the screen 100a of the external monitor 100 than the content of the display on the screen of the display operating part 10 at hand.

The display content controller 25 replaces a captured image on which a reverse process corresponding to the specific image-capture site has been performed with specific site correspondence image information. Specifically, in the case where a captured image of the five-image method of the standard imaging is acquired, site correspondence image information indicating the button corresponding to the site 1 (front face) in the oral cavity among the specific site setter 81 shown in FIG. 8A is replaced with a captured image of the front face on which the vertical reverse process and the lateral reverse process have been performed, and the captured image is then displayed on the screen of the display operating part 10. In addition, the same image is also displayed on the screen 100a of the external monitor 100.

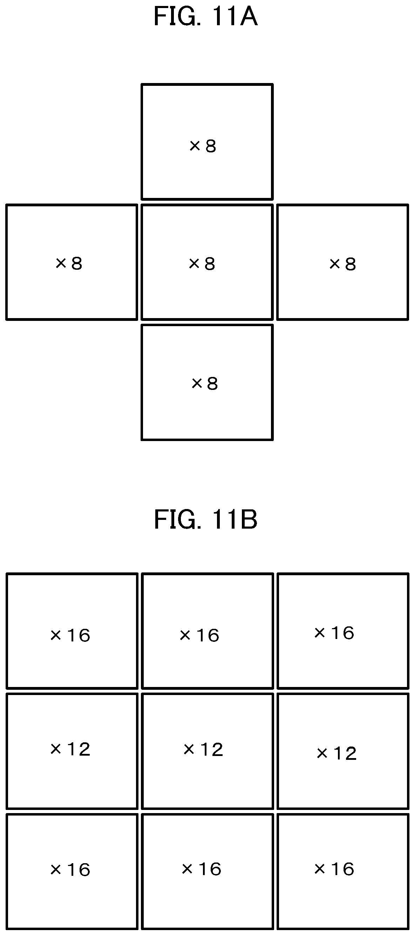

The image magnification factor adjuster 26 adjusts the magnification factor of a captured image depending on an image-capture site of the five-image method or the nine-image method for each imaging in the standard-imaging mode. The magnification factor of a captured image in the five-image method of the standard imaging is assumed to be 8-fold as shown in FIG. 11A. The magnification factor of a captured image in the nine-image method of the standard imaging is assumed to be 12-fold or 16-fold depending on an image-capture site as shown in FIG. 11B.

In response to information indicating an image-capture site inputted to the specific site setter 81 through the operation of the user, the image magnification factor adjuster 26 adjusts the magnification factor of a captured image to be acquired with respect to the image-capture site. For example, in the case where the nine-image method has been selected and the site 1 (front face) in the oral cavity has been selected in the specific site setter 81 of the display operating part 10, the image magnification factor adjuster 26 adjusts the magnification factor of the captured image with respect to the selected image-capture site to 12-fold.

In addition, in response to the specific site information indication region 71 indicated, the image magnification factor adjuster 26 adjusts the magnification factor of a captured image to be acquired by the standard-imaging assistance device 1.

For example, in the case where the nine-image method has been selected and the image-capture site determiner 23 has determined that the image-capture site is the site 4 (expanded maxillary occlusal surface) in the oral cavity, a region corresponding to the site 4 in the oral cavity is identifiably indicated in the specific site information indication region 71 of the display operating part 10. In this case, the image magnification factor adjuster 26 adjusts the magnification factor of a captured image with respect to an image-capture region corresponding to the site 4 in the oral cavity to 16-fold.

The image-capture controller 27 conducts control of causing the image capturer 2 to capture an image of an image-capture subject through the operation of the user. The image-capture controller 27 outputs control signals to the image capturer 2, the captured image generator 3, the captured image outputter 4, and the storage device 6 to control the operations of the image capturer 2, the captured image generator 3, the captured image outputter 4, and the storage device 6.

### [Flow of Processing of Standard-Imaging Mode]

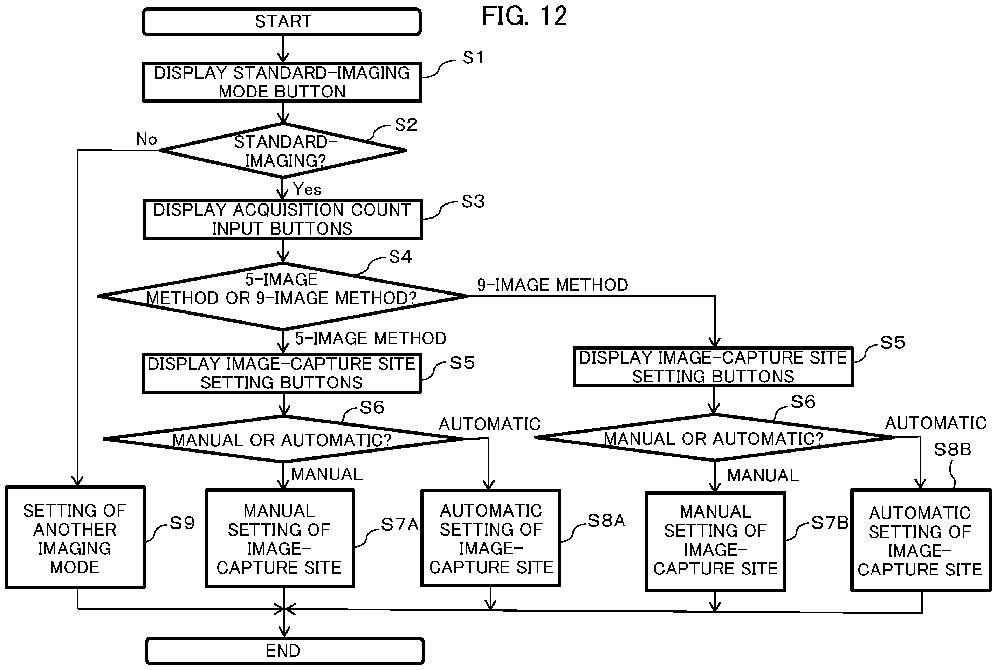

Next, a flow of processing of the standard-imaging mode of the standard-imaging assistance device 1 will be described with reference to FIG. 12 (with reference to FIG. 1 and FIG. 7A to FIG. 7C as appropriate). In the standard-imaging assistance device 1, imaging mode selector 22 of the controller 20 causes the display operating part 10 to display the standard-imaging mode button B02 (step S1), and determines whether or not an instruction to execute the standard imaging has been inputted (step S2). If an instruction to execute a mode other than the standard imaging has been inputted (step S2: No), the imaging mode selector 22 conducts the setting of another imaging mode (step S9). The other imaging mode indicates, for example, a normal imaging mode or the like.

On the other hand, when the standard-imaging mode button B02 is operated by the user (step S2: Yes), the imaging mode selector 22 causes the display operating part 10 to display the acquisition count input buttons B03 and B04 (step S3) and determines which button of the acquisition count input buttons B03 and B04 has been operated (step S4). When either of the acquisition count input buttons B03 and B04 is operated by the user, the imaging mode selector 22 causes the display operating part 10 to display the image-capture site setting buttons B05 and B06 (step S5). Then, the imaging mode selector 22 determines which button of the image-capture site setting buttons B05 and B06 has been operated (step S6).

If the image-capture site setting button B06 to instruct the manual setting is operated when the five-image method has been selected by the user, for example, the imaging mode selector 22 conducts the manual setting of an image-capture site of the five-image method (step S7A). In addition, if the image-capture site setting button B06 to instruct the manual setting is operated when the nine-image method has been selected, the imaging mode selector 22 conducts the manual setting of an image-capture site of the nine-image method (step S7B). The details of these steps S7A and S7B will be described later.

On the other hand, if the image-capture site setting button B05 to instruct the automatic setting is operated when the five-image method has been selected by the user, the imaging mode selector 22 conducts the automatic setting of an image-capture site of the five-image method (step S8A). In addition, if the image-capture site setting button B05 to instruct the automatic setting is operated when the nine-image method has been selected, the imaging mode selector 22 conducts the automatic setting of an image-capture site of the nine-image method (step S8B). The details of these steps S8A and S8B will be described later.

### (Manual Setting of Image-Capture Site)

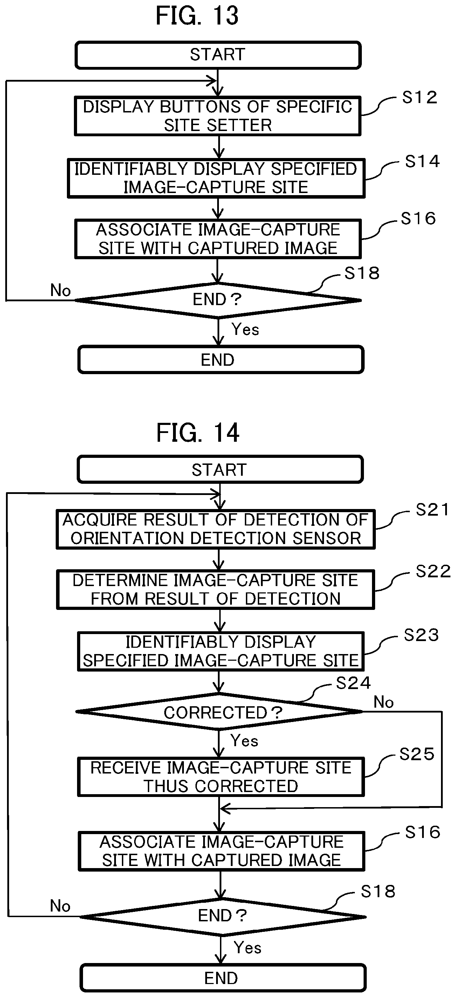

Next, the flow of step S7A of FIG. 12 will be described with reference to FIG. 13 (with reference to FIG. 1 as well as FIG. 8A and FIG. 8B as appropriate). The imaging mode selector 22 causes the display operating part 10 to display five buttons as the specific site setter 81 (step S12). Once the user operates a desired button from among the specific site setter 81 of the display operating part 10 to specify an image-capture site, the imaging mode selector 22 causes the display operating part 10 to identifiably display a button corresponding to the specified image-capture site (step S14). Then, once the user captures an image of the image-capture site of the examination subject, the imaging mode selector 22 associates the image-capture site with the captured image at this time (step S16).

Note that the standard-imaging assistance device 1 performs a reverse process corresponding to the image-capture site on the captured image through the processing of the controller 20 (the reverse pattern determiner 24 and the display content controller 25) and the captured image outputter 4 and then displays the captured image on which the reverse process has been performed on the display operating part 10, for example.

Then, the imaging mode selector 22 determines whether or not a predetermined ending condition has been satisfied (step S18). For example, in the case where the image-capture sites of the five-image method are associated with five captured images or in the case where an instruction to end the imaging is inputted, the ending condition is satisfied. If the ending condition has not been satisfied (step S18: No), the imaging mode selector 22 returns to step S12. If the ending condition has been satisfied (step S18: Yes), the imaging mode selector 22 ends the setting of image-capture sites.

On the other hand, the flow of the manual setting of an image-capture site of the nine-image method (step S7B) is similar to the flow shown in FIG. 13 and the description thereof is omitted. Note that in the manual setting of an image-capture site of the nine-image method, nine buttons are displayed on the display operating part 10 as buttons of the specific site setter 81.

### (Automatic Setting of Image-Capture Site)

Next, the flow of step S8A of FIG. 12 will be described with reference to FIG. 14 (with reference to FIG. 1 as well as FIG. 8C and FIG. 8D as appropriate). In the standard-imaging assistance device 1, the image-capture site determiner 23 of the controller 20 acquires a result of detection of the orientation detection sensor 5 (step S21), determines an image-capture site from the result of detection of the orientation detection sensor 5 (step S22), and outputs information of the determined image-capture site to the imaging mode selector 22. Note that the image-capture site determiner 23 stores a result of detection of the orientation detection sensor 5 acquired at the time of imaging the front face as a reference value.

Then, the imaging mode selector 22 causes the display operating part 10 to display five regions as the specific site information indication region 71 and to identifiably display a region of the image-capture site specified from the result of detection of the orientation detection sensor 5 (step S23). If the user has looked at the specific site information indication region 71 and corrected the specified image-capture site through manual operation (step S24: Yes), the imaging mode selector 22 receives the image-capture site thus corrected (step S25), and associates the corrected image-capture site with the captured image (step S16).

On the other hand, if the user has not corrected the specified image-capture site (step S24: No), the imaging mode selector 22 proceeds to the above-described step S16 and associates the specified image-capture site with the captured image. Then, the imaging mode selector 22 determines whether or not the predetermined ending condition has been satisfied (step S18). If the ending condition has not been satisfied (step S18: No), the standard-imaging assistance device 1 returns to step S21. If the ending condition has been satisfied (step S18: Yes), the standard-imaging assistance device 1 ends the setting of image-capture sites.

On the other hand, the flow of the automatic setting of an image-capture site of the nine-image method (step S8B) is similar to the flow shown in FIG. 14 and the description thereof is omitted. Note that in the automatic setting of an image-capture site of the nine-image method, nine regions are displayed on the display operating part 10 as the specific site information indication region 71.

### (Second Embodiment)

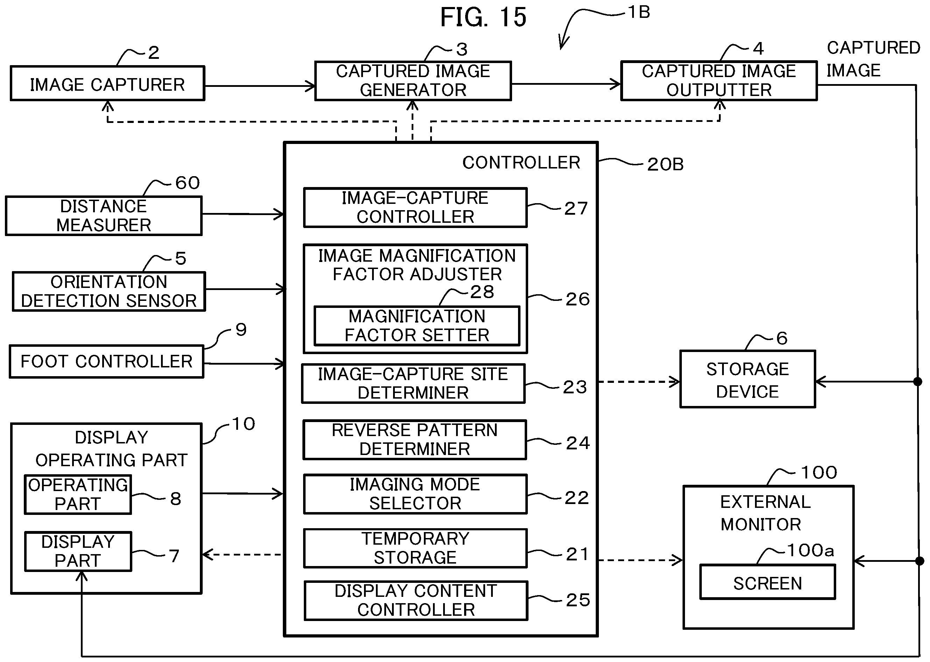

Next, a standard-imaging assistance device 1B according to a second embodiment will be described with reference to FIG. 15. Note that the same configurations as those of the standard-imaging assistance device 1 shown in FIG. 1 are denoted by the same signs and the description thereof is omitted. The standard-imaging assistance device 1B is different from the standard-imaging assistance device 1 in that the standard-imaging assistance device 1B includes a distance measurer 60 and includes a magnification factor setter 28 in a controller 20B.

The distance measurer 60 measures a distance between the standard-imaging assistance device 1B and an image-capture subject. Note that the expression "measure a distance" encompasses not only measuring an actual distance between the standard-imaging assistance device 1B and the image-capture subject but also estimating a distance between the standard-imaging assistance device 1B and the image-capture subject through image recognition, for example.

The distance measurer 60 refers to, for example, a distance measurement sensor (of an ultrasonic wave method or a laser light method), image recognition means (a camera, an image processor, a controller, or the like), or an AF (autofocus) mechanism (including a controller), or the like.

In the case where the distance measurer 60 is a distance measurement sensor, the distance between the standard-imaging assistance device 1 and the image-capture subject is a distance between the distance measurement sensor and the image-capture subject.

In the case where the distance measurer 60 is image recognition means, the distance between the standard-imaging assistance device 1 and the image-capture subject is a distance between a lens of a camera and the image-capture subject.

In the case where the distance measurer 60 is an AF mechanism (including a controller), the distance between the standard-imaging assistance device 1 and the image-capture subject refers to a distance between a lens of a camera and the image-capture subject, or a distance between an AF sensor and the image-capture subject, or the like.

The distance measurer 60 outputs the measured distance to the image magnification factor adjuster 26 (the magnification factor setter 28).

In the present embodiment, the distance measurer 60 is assumed to be a distance measurement sensor, and the distance measurement sensor has a function of irradiating the image-capture subject with color light. By causing the distance measurement sensor to point-display an image-capture portion with color light, the user is allowed to easily grasp an optical-axis direction of the distance measurer 60 (an emission direction of the color light), that is, an optical-axis direction of the image capturer 2 only by observing the inside of the oral cavity of the examination subject without checking the screen of the display operating part 10 or the screen 100a of the external monitor 100. The color light emitted from the distance measurement sensor is, for example, laser light or LED light. Note that the distance measurement sensor may be configured with a dedicated semiconductor IC chip, for example.

As shown in FIG. 15, the image magnification factor adjuster 26 of the controller 20B includes the magnification factor setter 28. In the present embodiment, the magnification factor setter 28 sets a magnification factor of a captured image to be acquired with respect to the image-capture subject based on a result of measurement of the distance measurer 60. Based on the result of measurement of the distance measurer 60, the magnification factor setter 28 corrects and sets a reference magnification factor such that the magnification factor of the captured image to be acquired with respect to the image-capture subject becomes the same even when the distance between the standard-imaging assistance device 1 and the image-capture subject varies. Here, the reference magnification factor is set in advance through the operation of the user. The captured image generator 3 adjusts the magnification factor of the captured image to be acquired with respect to the image-capture subject by using the magnification factor set by the magnification factor setter 28.

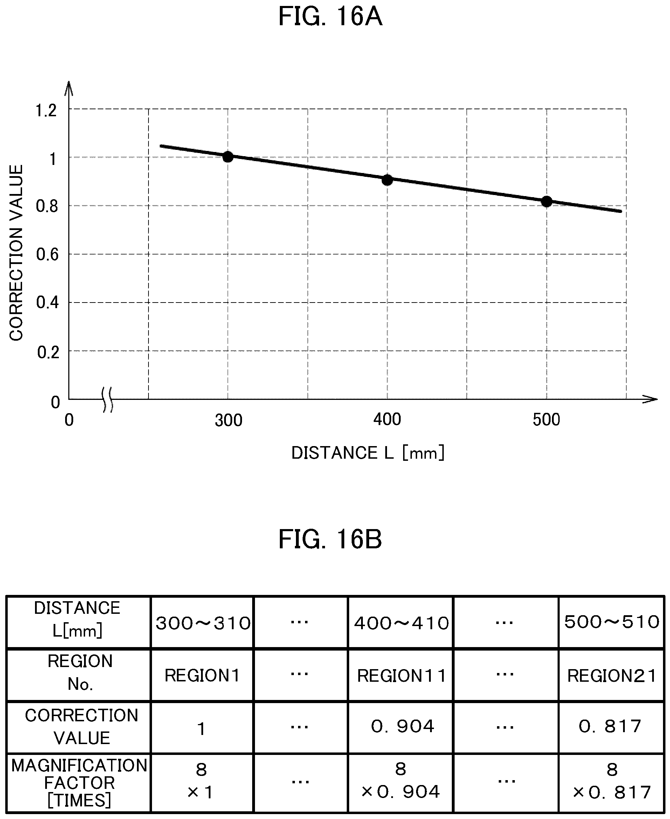

A specific example in which the magnification factor setter 28 corrects and sets a magnification factor of a captured image will be described with reference to FIG. 16A and FIG. 16B. FIG. 16A shows correspondence relation between distances and correction values in the case of imaging the site 1 (front face) in the oral cavity while setting the distance between the standard-imaging assistance device 1 and the imaging subject to 300 mm, 400 mm, and 500 mm. In this example, it is assumed that the imageable distance of the image capturer 2 is 300 to 500 mm, and the reference magnification factor is 8-fold. The magnification factor setter 28 sets a result of multiplying the reference magnification factor (for example, 8-fold) by a correction value as a magnification factor. Note that the correction value when the imageable distance of the image capturer 2 is the minimum value (300 mm) is set to 1 (reference value).

From the graph of FIG. 16A, it can be seen that a relational expression between the magnification factor to be set and the distance between the standard-imaging assistance device 1 and the image-capture subject is expressed by a linear function in which the value linearly decreases as the distance between the standard-imaging assistance device 1 and the image-capture subject increases. Hence, for example, the magnification factor setter 28 calculates a magnification factor through calculation using this linear function every time an image is captured.

Note that the magnification factor setter 28 is not limited to setting a magnification factor by using the above linear function. For example, a distance range which can be measured by the distance measurer 60 may be divided to set a plurality of regions, and a magnification factor for one region may be set for each of the plurality of regions such that the magnification factor decreases as the distance between the standard-imaging assistance device 1 and the image-capture subject increases. In this case, the magnification factor setter 28 sets a magnification factor for the captured image to be acquired with respect to the image-capture subject, by using one of the regional magnification factors based on the result of measurement of the distance measurer 60. In addition, in the case where regional magnification factors are set in a regional magnification factor table, the magnification factor setter 28 sets the magnification factor of the captured image for the image-capture subject by referring to the regional magnification factor table in which the regional magnification factors are stored in advance.

FIG. 16B is a diagram showing the regional magnification factor table. In this regional magnification factor table, regional magnification factors are stored in advance while the imageable distance is divided into regions of 10 mm each. In this example, it is assumed that the imageable distance of the image capturer 2 is 300 to 500 mm and the reference magnification factor is 8-fold. In this regional magnification factor table, the regional magnification factor of a region 1 (in the case where the distance L is 300 to 310 mm) is 8×1. The regional magnification factor of a region 11 (in the case where the distance Lis 400 to 410 mm) is 8×0.904. The regional magnification factor of a region 21 (in the case where the distance L is 500 to 510 mm) is 8×0.817.

Although the magnification factor of the captured image for the site 1 (front face) in the oral cavity has been described here, the magnification factors of the captured images of the other image-capture sites can be set in the same manner.

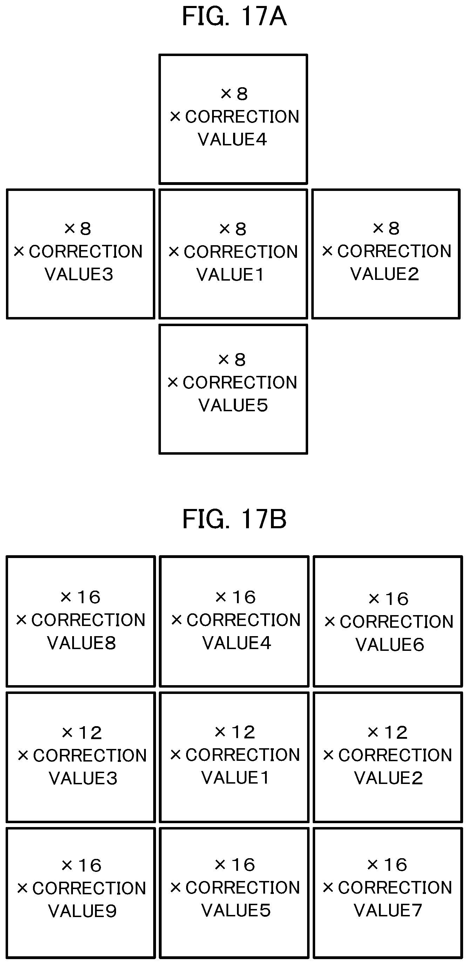

The image magnification factor adjuster 26 adjusts a magnification factor of a captured image based on the reference magnification factor depending on the image-capture site. It is assumed that in the standard imaging, the reference magnification factor of a captured image of the five-image method is 8-fold as shown in FIG. 17A. It is assumed that in the standard imaging, the reference magnification factor of a captured image of the nine-image method is 12-fold or 16-fold depending on the image-capture site as shown in FIG. 17B.

The magnification factor setter 28 uses a correction value depending on the image-capture site in order to correct the reference magnification factor depending on the image-capture site. Correction value 1 to correction value 5 shown in FIG. 17A are correction values for site 1 to site 5 in the oral cavity. In addition, in the case of the nine-image method, correction value 1 to correction value 9 shown in FIG. 17B are correction values for site 1 to site 9 in the oral cavity.

Note that in a simplified manner, correction value 1 to correction value 5 shown in FIG. 17A may be the same value, that is, a predetermined value α. In addition, correction value 1 to correction value 3 shown in FIG. 17B may be the same value, that is, a predetermined value β. Similarly, correction value 4 to correction value 9 shown in FIG. 17B may be the same value, that is, a predetermined value γ.

The conventional standard imaging has several problems. Since the conventional standard imaging employs an imaging method in which a camera such as a single-lens reflex camera is used and the shutter button of the camera is pressed when the teeth of an imaging subject are taken into the frame, it is difficult to standardize a magnification factor among a plurality of captured images. In addition, the adjustment of a magnification factor needs to be conducted for every imaging, and the work is cumbersome for a photographer. In addition, it is necessary to complete imaging each image-capture site within several minutes at most so as not to cause a burden on the patient. Moreover, even when a dentist arranges a plurality of captured images captured in this way in accordance with the five-image method or the nine-image method to grasp the state in the oral cavity of the examination subject, there is a case where the magnification factors of the captured images became uneven. That is, it is difficult to make the qualities of intraoral photographs a predetermined quality or more. In addition, even in the case where imaging is conducted with the magnification factor of the captured images being fixed in advance, since the captured images need to be captured such that the entire site to be an image-capture subject is taken into a frame, it is not easy to make the magnification factor even among the captured images in this point as well.

In contrast, since the standard-imaging assistance device 1B according to the second embodiment corrects and sets the magnification factor of each captured image in accordance with the distance between the standard-imaging assistance device 1B and the image-capture subject, it is possible to reduce the burden of the work to standardize the magnification factor among a plurality of captured images.

Note that although the embodiments of the present invention have been described in detail, the present invention is not limited to the above-described embodiments and encompasses those obtained by modifying the embodiments in design without departing from the gist of the present invention, and the like. Although the distance measurer 60 is described as a distance measurement sensor which irradiates an image-capture subject with color light, a configuration that includes no distance measurement sensor may be employed, or a configuration that has no distance measurement sensor may be employed. Having no distance measurement sensor means that a distance measurement sensor can be attached but has been removed. In the case of a configuration in which the standard-imaging assistance device 1 includes or has no distance measurement sensor, an AF function may be utilized as a substitute for a distance measurement sensor, or a distance to an image-capture subject may be estimated through image recognition.

In the case where the standard-imaging assistance device 1B does not have the distance measurer 60, the image capturer 2 may capture an image with an image pickup range made wider than the image pickup range of captured images used in the standard imaging in advance. In this case, the image magnification factor adjuster 26 may perform a trimming process on a captured image which is captured by the image capturer 2 with an image pickup range made wider than the image pickup range of captured images used in the standard imaging in advance.

This makes it possible to reduce the burden of the work to standardize the magnification factor among a plurality of captured images even without the distance measurer 60.

Moreover, when the image magnification factor adjuster 26 performs the trimming process on a captured image which is captured by the image capturer 2 with an image pickup range made wider than the image pickup range of captured images used in the standard imaging in advance, the magnification factor setter 28 of the image magnification factor adjuster 26 may set the magnification factor as follows. Specifically, the magnification factor setter 28 may set the magnification factor of a captured image to be acquired with respect to the image-capture subject such that the image pickup range becomes wider than the image pickup range of captured images used in the standard imaging.

In the case of determining an image-capture site of the nine-image method, the image-capture site determiner 23 may determine the image-capture site as follows after images of image-capture sites corresponding to the center portion and upper, lower, left, and right portions in the matrix of FIG. 9B are captured. In the case of capturing images of image-capture sites corresponding to the four corner portions of the matrix, if a result of detection of the orientation detection sensor 5 has even slightly inclined from the reference position, the image-capture site determiner 23 may specify an image-capture site corresponding to a corner located in the direction of the inclination. Specifically, for example, if the result of detection has even slightly inclined to the upper right side, the image-capture site determiner 23 may determine that the image-capture site is located at the right upper side among the four corners the image of which have not been captured.

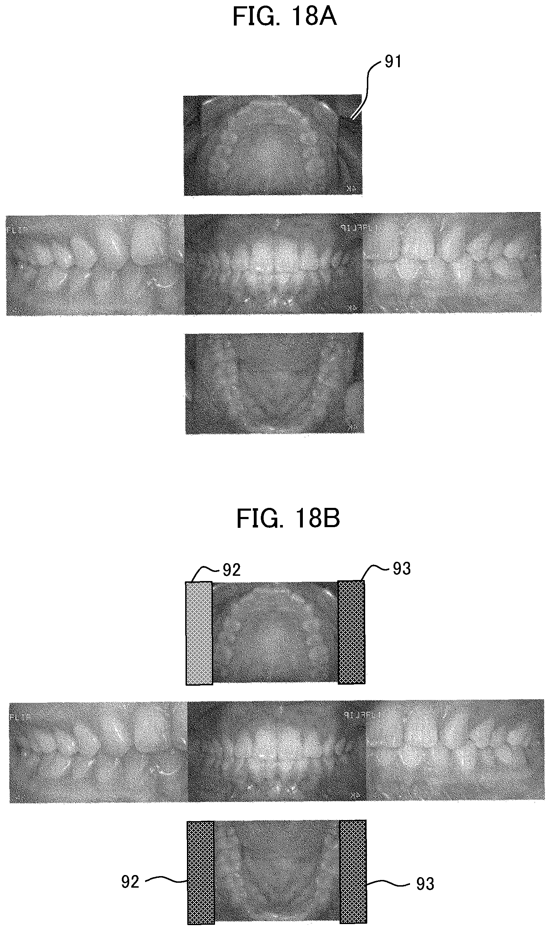

As shown in FIG. 18A, in the standard imaging, there is a case where a mirror 91 is taken into a captured image. In view of this, the captured image outputter 4 may perform a masking process on a portion where the mirror 91 is likely to be taken in a captured image. In this case, as shown in FIG. 18B, the captured image outputter 4 performs a masking process on a left end portion 92 or a right end portion 93 of, for example, a captured image of the site 4 (the maxillary occlusal surface) or a captured image of the site 5 (the mandibular occlusal surface) in the oral cavity. The masking process only has to be capable of hiding a portion where an edge portion of the mirror 91 is likely to be taken in, and may be, for example, a process of painting the portion with a color similar to the color of the inside of the oral cavity or an unnoticeable color. Here, the color similar to the color of the inside of the oral cavity is, for example, red, a skin color, or the like, and the unnoticeable color is, for example, gray or the like.

Although the standard-imaging assistance devices 1 and 1B have been described to include the display operating part 10, the display part 7 does not necessarily have to be a liquid-crystal display panel, and the operating part 8 does not necessarily have to be a touch panel. In the case where the buttons of the operating part 8 are not the buttons displayed on a touch panel, the buttons may be, for example, mechanical switches (including a sheet switch). In the case where the buttons of the operating part 8 are mechanical switches, the operation may be such that all the steps of displaying the buttons are deleted in the flowcharts of FIG. 12 and FIG. 13. In the case where the buttons of the operating part 8 are mechanical switches or buttons displayed on a curved display, even when five buttons included in the specific site setter 81 are provided on a curved surface, these five buttons only have to be arranged to be seen in a cross shape as viewed from the user.

The specific site information indication region 71 has been described to be displayed on the screen of the display operating part 10, but is not limited to this. The specific site information indication region 71 may be, for example, composed of nine LED lamps or the like arranged in a matrix shape of vertical three columns and horizontal three rows corresponding to the image-capture sites of the nine-image method. In this case, in the case where information of an image-capture site the image of which is to be captured is indicated from the image-capture site determiner 23, the imaging mode selector 22 identifiably turns on the LED lamp corresponding to the image-capture site in the specific site information indication region 71.

In the case of automatically setting an image-capture site of the five-image method or the nine-image method, the standard-imaging assistance device 1, 1B may indicate, with voice, which image-capture site of a plurality of image-capture sites in the oral cavity of the examination subject, the image-capture site of the captured image to be acquired by the standard-imaging assistance device 1, 1B is.

In the above embodiments, the standard-imaging assistance device 1, 1B includes the image capturer 2, the captured image generator 3, the captured image outputter 4, the orientation detection sensor 5, the storage device 6, the display part 7, and the foot controller 9, but all of these are not essential configurations. If the standard-imaging assistance device 1, 1B includes at least the operating part 8 and the controller 20, 20B, since the standard-imaging assistance device 1, 1B executes the standard-imaging mode in accordance with a setting operation received in the operating part 8, it is possible to make the standard imaging easier than the conventional technique.

In addition, as long as the standard-imaging assistance device 1, 1B includes the operating part 8 and the controller 20, 20B, the image capturer 2 may be separated, and for example, the standard-imaging assistance device 1, 1B may be configured with a personal computer to which the image capturer 2 is connected. In this case, the controller 20, 20B of the personal computer acquires a captured image from the image capturer 2 and executes the standard-imaging mode in accordance with a setting operation received by the operating part 8 such as a mouse or a keyboard, for example.

Hereinafter, various aspects of the present disclosure will be described collectively as Appendices.

### (Appendix 1)

A standard-imaging assistance device which includes an imaging mode of standard imaging to image an inside of an oral cavity of an examination subject and which assists the standard imaging conducted by a user, comprising:
an operating part which receives a setting operation of an imaging mode from the user; and
a controller which captures images of a plurality of image-capture sites specified in the oral cavity of the same examination subject by using an image capturer in accordance with the setting operation received in the operating part and executes a standard-imaging mode which associates the captured images thus captured with one another.

### (Appendix 2)

The standard-imaging assistance device according to Appendix 1, wherein
the operating part has a standard-imaging mode button with which it is possible to input a standard-imaging mode execution instruction for executing the standard-imaging mode.

### (Appendix 3)

The standard-imaging assistance device according to Appendix 1 or 2, wherein
the operating part has an acquisition count input button with which it is possible to input whether to execute a five-image method acquiring mode in which a captured image of a five-image method of standard imaging is acquired or a nine-image method acquiring mode in which a captured image of a nine-image method of standard imaging is acquired.

### (Appendix 4)

The standard-imaging assistance device according to Appendix 1, 2, or 3, further comprising:
a sensor which detects a three-dimensional orientation of the image capturer relative to a reference orientation of the image capturer, wherein
the controller includes an image-capture site determiner which determines which image-capture site of the plurality of image-capture sites, an image-capture site of a captured image to be acquired by the standard-imaging assistance device is, based on a result of detection of the sensor.

### (Appendix 5)

The standard-imaging assistance device according to Appendix 4, wherein
the operating part has a specific site information indication region for indicating a captured image to be acquired by the standard-imaging assistance device or indicating which image-capture site of the plurality of image-capture sites, an image-capture site of the captured image is.

### (Appendix 6)

The standard-imaging assistance device according to Appendix 5, wherein
the specific site information indication region is composed of five regions corresponding respectively to image-capture sites of a five-image method of standard imaging, and
the five regions are arranged in a cross shape.

### (Appendix 7)

The standard-imaging assistance device according to Appendix 5, wherein
the specific site information indication region is composed of nine regions corresponding respectively to image-capture sites of a nine-image method of standard imaging, and
the nine regions are arranged in a matrix shape of vertical three columns and horizontal three rows.

### (Appendix 8)

The standard-imaging assistance device according to Appendix 1, 2, or 3, wherein
the operating part has a specific site setter with which it is possible to input which image-capture site of the plurality of image-capture sites, an image-capture site of a captured image to be acquired by the standard-imaging assistance device is.

### (Appendix 9)

The standard-imaging assistance device according to Appendix 8, wherein
the specific site setter is composed of five buttons corresponding respectively to image-capture sites of a five-image method of standard imaging, and
the five buttons are arranged in a cross shape.

### (Appendix 10)

The standard-imaging assistance device according to Appendix 8, wherein
the specific site setter is composed of nine buttons corresponding respectively to image-capture sites of a nine-image method of standard imaging, and
the nine buttons are arranged in a matrix shape of vertical three columns and horizontal three rows.

### (Appendix 11)

The standard-imaging assistance device according to Appendix 8, 9, or 10, wherein
the controller includes:
an image-capture controller which conducts control of causing the image capturer to capture an image of an image-capture subject through an operation of the user; and
an image magnification factor adjuster which adjusts, in response to information indicating an image-capture site inputted to the specific site setter through an operation of the user, a magnification factor of a captured image to be acquired with respect to the image-capture site.

### (Appendix 12)

The standard-imaging assistance device according to Appendix 5, 6, or 7, wherein
the controller includes:
an image-capture controller which conducts control of causing the image capturer to capture an image of an image-capture subject through an operation of the user; and
an image magnification factor adjuster which adjusts, in response to the indicated specific site information indication region, a magnification factor of the captured image to be acquired.

### (Appendix 13)

The standard-imaging assistance device according to Appendix 4, 5, 6, 7, or 12, wherein
the operating part has an image-capture site setting button with which it is possible to input whether to automatically set or manually set which image-capture site of the plurality of image-capture sites, an image-capture site of a captured image to be acquired by the standard-imaging assistance device is.

### (Appendix 14)

The standard-imaging assistance device according to Appendix 11 or 12, further comprising:
a distance measurer which measures a distance between the standard-imaging assistance device and an image-capture subject, wherein
the image magnification factor adjuster has a magnification factor setter which sets a magnification factor of a captured image to be acquired with respect to the image-capture subject based on a result of measurement of the distance measurer.

### (Appendix 15)

The standard-imaging assistance device according to Appendix 11 or 12, wherein
the image magnification factor adjuster performs a trimming process on a captured image which is captured by the image capturer with an image pickup range made wider than an image pickup range of a captured image used in standard imaging in advance.

### (Appendix 16)

The standard-imaging assistance device according to Appendix 15, wherein
the image magnification factor adjuster has a magnification factor setter which sets a magnification factor of a captured image to be acquired with respect to the image-capture subject such that an image pickup range becomes wider than the image pickup range of the captured image used in the standard imaging.

### (Appendix 17)

The standard-imaging assistance device according to Appendix 14 or 16, further comprising:
the image capturer; and
a captured image generator which generates a captured image based on an image signal outputted from the image capturer, wherein
the captured image generator adjusts a magnification factor of a captured image to be acquired with respect to the image-capture subject by using a magnification factor set by the magnification factor setter.

### (Appendix 18)

The standard-imaging assistance device according to Appendix 9 or 10, further comprising:
a display operating part which is configured by integrating the operating part and a display part which displays a captured image to be acquired by the standard-imaging assistance device or a captured image acquired by the standard-imaging assistance device on a screen, wherein
each of buttons included in the specific site setter is specific site correspondence image information displayed on the screen of the display operating part.

### (Appendix 19)

The standard-imaging assistance device according to Appendix 6 or 7, further comprising:
a display operating part which is configured by integrating the operating part and a display part which displays a captured image to be acquired by the standard-imaging assistance device or a captured image acquired by the standard-imaging assistance device on a screen, wherein
each of regions included in the specific site information indication region is specific site correspondence image information displayed on the display operating part.

### (Appendix 20)

The standard-imaging assistance device according to Appendix 18 or 19, wherein
the controller includes a display content controller which controls a content of display on the screen of the display operating part, and
the display content controller replaces a captured image with respect to a specific image-capture site acquired by the image capturer with the specific site correspondence image information corresponding to the image-capture site and causes the display operating part to display the specific site correspondence image information.

### (Appendix 21)

The standard-imaging assistance device according to Appendix 18 or 19, wherein
the controller includes a display content controller which controls a content of display on a screen of a connected external monitor, and
the display content controller replaces a captured image with respect to a specific image-capture site acquired by the image capturer with the specific site correspondence image information corresponding to the image-capture site and causes the screen of the external monitor to display the specific site correspondence image information.

### (Appendix 22)

The standard-imaging assistance device according to Appendix 20 or 21, wherein
the display content controller replaces a captured image on which a reverse process corresponding to a specific image-capture site has been performed with the specific site correspondence image information.

### (Appendix 23)

The standard-imaging assistance device according to Appendix 14, wherein
the magnification factor setter corrects a reference magnification factor set in advance through an operation of the user such that even when a distance between the standard-imaging assistance device and the image-capture subject varies, a magnification factor of a captured image to be acquired with respect to the image-capture subject becomes the same based on a result of measurement of the distance measurer and sets the magnification factor of the captured image to be acquired with respect to the image-capture subject.

### (Appendix 24)

The standard-imaging assistance device according to Appendix 23, wherein
a distance range which can be measured by the distance measurer is divided to set a plurality of regions,
a magnification factor for one region is set for each of the plurality of regions such that the magnification factor decreases as the distance between the standard-imaging assistance device and the image-capture subject increases,
the magnification factor setter sets a magnification factor of a captured image to be acquired with respect to the image-capture subject, by using one of the regional magnification factors based on a result of measurement of the distance measurer.

### (Appendix 25)

The standard-imaging assistance device according to Appendix 24, wherein
the magnification factor setter sets a magnification factor of a captured image to be acquired with respect to the image-capture subject by referring to a regional magnification factor table in which the regional magnification factors are stored in advance.

### (Appendix 26)

The standard-imaging assistance device according to Appendix 23, wherein
a relational expression between the magnification factor to be set and the distance between the standard-imaging assistance device and the image-capture subject is expressed by a linear function in which a value linearly decreases as the distance between the standard-imaging assistance device and the image-capture subject increases, and
the magnification factor setter calculates the magnification factor to be set through calculation using the linear function when an image is captured.

### (Appendix 27)

The standard-imaging assistance device according to Appendix 9, 10, or 11, further comprising:
a reverse pattern determiner which determines, in response to an image-capture site inputted to the specific site setter through an operation of the user, which of a plurality of reverse processes prepared in advance, a reverse process to be performed on a captured image acquired from the image-capture site as an image-capture subject is; and
a captured image outputter which performs the reverse process on the acquired captured image based on a result of the determination of the reverse pattern determiner and outputs the captured image on which the reverse process has been performed.

### (Appendix 28)

The standard-imaging assistance device according to Appendix 4, further comprising:
a reverse pattern determiner which determines, in response to an image-capture site determined by the image-capture site determiner, which of a plurality of reverse processes prepared in advance, a reverse process to be performed on a captured image acquired from the image-capture site as an image-capture subject is; and
a captured image outputter which performs the reverse process on the acquired captured image based on a result of the determination of the reverse pattern determiner and outputs the captured image on which the reverse process has been performed.

### (Appendix 29)

The standard-imaging assistance device according to Appendix 27 or 28, wherein
the captured image outputter outputs data of the captured image on which the reverse process has been performed in a format containing information on an image-capture site of an image-capture subject from which the captured image has been acquired.

### (Appendix 30)

The standard-imaging assistance device according to Appendix 27, 28, or 29, further comprising:
a storage device which stores data of the outputted captured image.

### (Appendix 31)

The standard-imaging assistance device according to Appendix 14, 23, 24, 25, or 26, wherein
the distance measurer is a distance measurement sensor, and
the distance measurement sensor has a function of irradiating the image-capture subject with color light.

### Reference Signs List

- 1, 1B: standard-imaging assistance device
- 2: image capturer
- 3: captured image generator
- 4: captured image outputter
- 5: orientation detection sensor
- 6: storage device
- 7: display part
- 8: operating part
- 9: foot controller
- 10: display operating part
- 20, 20B: controller
- 21: temporary storage
- 22: imaging mode selector
- 23: image-capture site determiner
- 24: reverse pattern determiner
- 25: display content controller
- 26, 26B: image magnification factor adjuster
- 27: image-capture controller
- 28: magnification factor setter
- 31: main body
- 32: grip part
- 60: distance measurer
- 71: specific site information indication region
- 81: specific site setter
- 100: external monitor
- 100a: screen
- B01: normal-imaging mode button
- B02: standard-imaging mode button
- B03, B04: acquisition count input button
- B05, B06: image-capture site setting button

## Claims

1. A standard-imaging assistance device which includes an imaging mode of standard imaging to image an inside of an oral cavity of an examination subject and which assists the standard imaging conducted by a user, comprising:
an operating part which receives a setting operation of an imaging mode from the user; and
a controller which captures images of a plurality of image-capture sites specified in the oral cavity of the same examination subject by using an image capturer in accordance with the setting operation received in the operating part and executes a standard-imaging mode which associates the captured images thus captured with one another.

2. The standard-imaging assistance device according to claim 1, wherein
the operating part has a standard-imaging mode button with which it is possible to input a standard-imaging mode execution instruction for executing the standard-imaging mode.

3. The standard-imaging assistance device according to claim 1 or claim 2, wherein
the operating part has an acquisition count input button with which it is possible to input whether to execute a five-image method acquiring mode in which a captured image of a five-image method of standard imaging is acquired or a nine-image method acquiring mode in which a captured image of a nine-image method of standard imaging is acquired.

4. The standard-imaging assistance device according to any one of claims 1 to 3, further comprising:
a sensor which detects a three-dimensional orientation of the image capturer relative to a reference orientation of the image capturer, wherein
the controller includes an image-capture site determiner which determines which image-capture site of the plurality of image-capture sites, an image-capture site of a captured image to be acquired by the standard-imaging assistance device is, based on a result of detection of the sensor.

5. The standard-imaging assistance device according to claim 4, wherein
the operating part has a specific site information indication region for indicating a captured image to be acquired by the standard-imaging assistance device or indicating which image-capture site of the plurality of image-capture sites, an image-capture site of the captured image is.

6. The standard-imaging assistance device according to any one of claims 1 to 3, wherein
the operating part has a specific site setter with which it is possible to input which image-capture site of the plurality of image-capture sites, an image-capture site of a captured image to be acquired by the standard-imaging assistance device is.

7. The standard-imaging assistance device according to claim 6, wherein
the controller includes:
an image-capture controller which conducts control of causing the image capturer to capture an image of an image-capture subject through an operation of the user; and
an image magnification factor adjuster which adjusts, in response to information indicating an image-capture site inputted to the specific site setter through an operation of the user, a magnification factor of a captured image to be acquired with respect to the image-capture site.

8. The standard-imaging assistance device according to claim 5, wherein
the controller includes:
an image-capture controller which conducts control of causing the image capturer to capture an image of an image-capture subject through an operation of the user; and
an image magnification factor adjuster which adjusts, in response to the indicated specific site information indication region, a magnification factor of the captured image to be acquired.

9. The standard-imaging assistance device according to any one of claims 4, 5, and 8, wherein
the operating part has an image-capture site setting button with which it is possible to input whether to automatically set or manually set which image-capture site of the plurality of image-capture sites, an image-capture site of a captured image to be acquired by the standard-imaging assistance device is.

10. The standard-imaging assistance device according to claim 7 or 8, further comprising:
a distance measurer which measures a distance between the standard-imaging assistance device and an image-capture subject, wherein
the image magnification factor adjuster has a magnification factor setter which sets a magnification factor of a captured image to be acquired with respect to the image-capture subject based on a result of measurement of the distance measurer.

11. The standard-imaging assistance device according to claim 7 or 8, wherein
the image magnification factor adjuster performs a trimming process on a captured image which is captured by the image capturer with an image pickup range made wider than an image pickup range of a captured image used in standard imaging in advance.

12. The standard-imaging assistance device according to claim 10, further comprising:
the image capturer; and
a captured image generator which generates a captured image based on an image signal outputted from the image capturer, wherein
the captured image generator adjusts a magnification factor of a captured image to be acquired with respect to the image-capture subject by using a magnification factor set by the magnification factor setter.

13. The standard-imaging assistance device according to claim 6, further comprising:
a display operating part which is configured by integrating the operating part and a display part which displays a captured image to be acquired by the standard-imaging assistance device or a captured image acquired by the standard-imaging assistance device on a screen, wherein
each of buttons included in the specific site setter is specific site correspondence image information displayed on the screen of the display operating part.

14. The standard-imaging assistance device according to claim 5, further comprising:
a display operating part which is configured by integrating the operating part and a display part which displays a captured image to be acquired by the standard-imaging assistance device or a captured image acquired by the standard-imaging assistance device on a screen, wherein
each of regions included in the specific site information indication region is specific site correspondence image information displayed on the display operating part.

15. The standard-imaging assistance device according to claim 13 or 14, wherein
the controller includes a display content controller which controls a content of display on the screen of the display operating part, and
the display content controller replaces a captured image with respect to a specific image-capture site acquired by the image capturer with the specific site correspondence image information corresponding to the image-capture site and causes the display operating part to display the specific site correspondence image information.

16. The standard-imaging assistance device according to claim 13 or 14, wherein
the controller includes a display content controller which controls a content of display on a screen of a connected external monitor, and
the display content controller replaces a captured image with respect to a specific image-capture site acquired by the image capturer with the specific site correspondence image information corresponding to the image-capture site and causes the screen of the external monitor to display the specific site correspondence image information.

17. The standard-imaging assistance device according to claim 15 or 16, wherein
the display content controller replaces a captured image on which a reverse process corresponding to a specific image-capture site has been performed with the specific site correspondence image information.

18. The standard-imaging assistance device according to claim 10, wherein
the magnification factor setter corrects a reference magnification factor set in advance through an operation of the user such that even when a distance between the standard-imaging assistance device and the image-capture subject varies, a magnification factor of a captured image to be acquired with respect to the image-capture subject becomes the same based on a result of measurement of the distance measurer and sets the magnification factor of the captured image to be acquired with respect to the image-capture subject.

19. The standard-imaging assistance device according to claim 7, further comprising:
a reverse pattern determiner which determines, in response to an image-capture site inputted to the specific site setter through an operation of the user, which of a plurality of reverse processes prepared in advance, a reverse process to be performed on a captured image acquired from the image-capture site as an image-capture subject is; and
a captured image outputter which performs the reverse process on the acquired captured image based on a result of the determination of the reverse pattern determiner and outputs the captured image on which the reverse process has been performed.

20. The standard-imaging assistance device according to claim 4, further comprising:
a reverse pattern determiner which determines, in response to an image-capture site determined by the image-capture site determiner, which of a plurality of reverse process prepared in advance, a reverse process to be performed on a captured image acquired from the image-capture site as an image-capture subject is; and
a captured image outputter which performs the reverse process on the acquired captured image based on a result of the determination of the reverse pattern determiner and outputs the captured image on which the reverse process has been performed.

21. The standard-imaging assistance device according to claim 19 or 20, further comprising:
a storage device which stores data of the outputted captured image.

22. The standard-imaging assistance device according to claim 10 or 18, wherein
the distance measurer is a distance measurement sensor, and
the distance measurement sensor has a function of irradiating the image-capture subject with color light.
